# EUROPÄISCHE PATENTSCHRIFT

(11) **EP 0 466 846 B1**
(45) Veröffentlichungstag und Bekanntmachung des Hinweises auf die Patenterteilung: **24.08.1994**
(21) Anmeldenummer: 91901609.7
(22) Anmeldetag: 17.01.1991
(51) Int. Cl.: G01N 21/89

(54) **VERFAHREN ZUR MESSUNG VON FASERPARAMETERN MITTELS BILDDATENVERARBEITUNG**
PROCESS FOR MEASURING FIBRE PARAMETERS BY IMAGE DATA PROCESSING
PROCEDE DE MESURE DE PARAMETRES DE FIBRES TEXTILES PAR TRAITEMENT DE DONNEES D'IMAGE

(30) Priorität: 02.02.1990 CH 343/90
(43) Veröffentlichungstag der Anmeldung: 22.01.1992
(73) Patentinhaber: ZELLWEGER LUWA AG, CH-8610 Uster (CH)
(72) Erfinder: GLOOR, René, CH-8805 Richterswil (CH)
(86) Internationale Anmeldenummer: CH9100017
(87) Internationale Veröffentlichungsnummer: WO9111705

(56) Entgegenhaltungen:
- EP-A- 0 009 999
- EP-A- 0 335 559
- GB-A- 2 148 498
- International Dyer & Textile Printer, August 1985, Industrial Press, (Old Trafford, Manchester, GB) "Inspection by laser", page 15

## Beschreibung

Die Erfindung bezieht sich auf ein Verfahren zur Messung von quantitativen und qualitativen Eigenschaften von Fasern, insbesondere von Textilfasern gemäss dem Oberbegriff des Anspruchs 1.

Es sind bereits Verfahren bekannt, mit denen die wichtigsten optisch messbaren Faserparameter, nämlich Länge, Feinheit, Reifegrad und Kräuselung bestimmt werden können.

Eines der bekanntesten Messgeräte für die Faserlänge ist das "Almeter" (Firma Siegfried Peyer AG), das die Längenverteilung eines endengeordneten Faserbartes misst. Ein Vorteil dieses Gerätes ist die korrekte Messung des Kurzfaseranteils. Die Messung erfolgt kapazitiv. Bei den Fasermessstrassen (High Volume-Instrumenten) wird mit einem Luftstrom oder mit einer Lichtintensitätsmessung die Länge der nicht endengeordneten Fasern bestimmt.

Die mittlere Feinheit von Baumwollfasern wird mit dem Luftstromverfahren bestimmt. Mit dem Schwingungsverfahren oder mit dem Mikroskop lässt sich die Feinheitsverteilung messen.

Die schnellste und damit verbreiteteste Methode, um den mittleren Reifegrad zu bestimmen, ist das leicht abgewandelte Luftstromverfahren der Feinheitsmessung. Die Geräte FMTII und FMTIII der Firma Shirley arbeiten nach diesem Prinzip. Ferner lässt sich der Reifegrad auch mit dem Mikroskop bestimmen.

Für die Messung der Kräuselung der Fasern gibt es noch keine weit verbreiteten Messgeräte. Es gibt aber Anleitungen und Normen wie die Kräuselung manuell gemessen werden kann.

Aus der CH-A5 661.118 ist auch bereits ein Verfahren gemäss dem Oberbegriff des Anspruchs 1 bekannt. Nachteilig bei diesem bekannten Verfahren ist seine Beschränkung auf die Längenmessung. Die darin verwendeten CCD-Sensoren und Kameras werden als zweidimensionales Einzelsensor-array eingesetzt, so dass nicht der Informationsgehalt des gesamten Sensors verwendet werden kann.

Aufgabe der Erfindung ist es ein Verfahren zu schaffen, mit dem die genaue, direkte und vollautomatische Messung von optisch erfassbaren Faserparametern möglich ist.

Die Erfindung löst die gestellte Aufgabe mit einem Verfahren, welches die Merkmale des Anspruchs 1 aufweist, sowie einer Vorrichtung zur Durchführung des erfindungsgemässen Verfahrens, welches die Merkmale des Anspruchs 7 aufweist.

Die durch die Erfindung erreichten Vorteile sind vielfältig und werden im einzelnen nachstehend aufgeführt:
- Bisher musste für jeden Faserparameter eine eigene Messmethode entwickelt werden. Dank des erfindungsgemässen Verfahrens lassen sich nun nebeneinander Längenverteilung, Feinheitsverteilung, Reifegradverteilung und Kräuselung bestimmen. Dies sind nur die wichtigsten Parameter. Es können natürlich noch weitere Faserparameter, die optisch erkennbar sind, gemessen werden. Der Entwicklungsaufwand beschränkt sich auf eine angepasste Abbildungsoptik und eine Auswertesoftware.
- Die Fasern werden direkt mit physikalischen Parametern gemessen. Dadurch ist auch eine einfache und genaue Eichung des Messgerätes möglich. Bei den bisherigen Messgeräten mussten vielfach indirekte Messmethoden verwendet werden.
- Das erfindungsgemässe Verfahren ist unabhängig von der Faserart, d.h. es können Baumwolle, Wolle, Man Made Fibers, Glasfasern, Drähte usw. gemessen werden. Nur die Optik und die Auswertealgorhythmen können davon betroffen werden.
- Da es sich nicht um eine integrale Messung handelt wie die bisherigen Methoden, können alle Parameter an einer Faser gemessen, bzw. einer spezifischen Faser zugeordnet werden. So ist es z.B. möglich eine Relation zwischen Länge, Dicke, Reife und Kräuselung pro Faser zu berechnen.
- Die Genauigkeit ist gleich derjenigen eines Mikroskopes.
- Die Reproduzierbarkeit ist besser als bei einem Mikroskop, da der Bedienereinfluss entfällt.
- Die Messgeschwindigkeit ist gegenüber dem Mikroskop um Faktoren grösser.
- Die aufgenommenen Bilder können ausgedruckt werden, wodurch eine physikalische Nachkontrolle der Resultate ohne technische Hilfsmittel möglich ist, was bei den bisherigen Messmethoden unmöglich war.
- An den exakt gleichen Faserbärten können mehrere verschiedene Parameter gemessen werden. Ebenso ist eine genaue Nachkontrolle der Messungen möglich, was bei bisherigen Messgeräten unmöglich ist, da jedes Messgerät wegen der verschiedenen Messprinzipien auch verschiedene Faservorbereitungen verlangt, weshalb für jede Messung ein neues Fasermuster erzeugt werden muss.
- Wenn die Rechenzeit für die Auswertung unwichtig ist, können auch Fasern gemessen werden, die als einlagiges wirres Vlies vorliegen. Die Auswertesoftware kann dann die Fasern separieren und verfolgen.

Gegenüber den Verfahren des Standes Technik verwendet das erfindungsgemässe Verfahren immer den Informationsgehalt des gesamten Sensors, der als ganzes verarbeitet werden kann. Deshalb können Störfaktoren bei der Längenmessung, wie z.B. Kräuselung, kreuzende Fasern, querliegende und nicht endengeordnete Fasern korrigiert und detektiert werden, wodurch die Genauigkeit des Verfahrens erheblich erhöht wird.

Ein Ausführungsbeispiel der Erfindung, welches zugleich das Funktionsprinzip erläutert, ist in der Zeichnung dargestellt und wird im folgenden näher beschrieben.

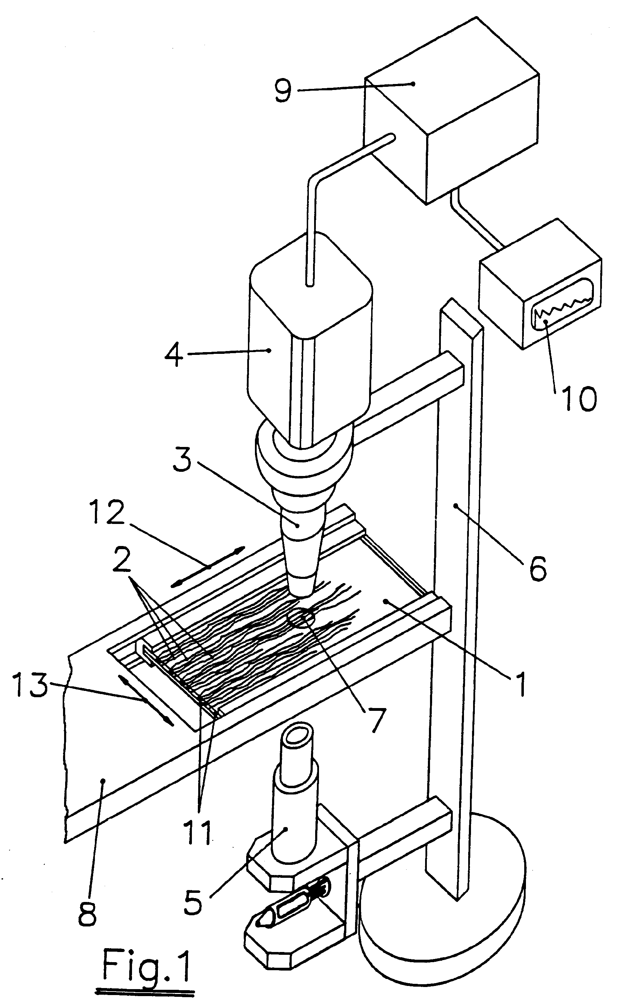

Fig. 1 zeigt eine perspektivische Darstellung eines Gerätes zur Durchführung des erfindungsgemässen Verfahrens.

Die in Fig. 1 dargestellte Vorrichtung zur Durchführung des erfindungsgemässen Verfahrens besteht grundsätzlich aus einer Faserzuführ-Einrichtung 1, einer Beleuchtungsoptik 5, einer Abbildungsoptik 3, einem CCD-Sensor 4, einem Rechner 9 und einem Anzeigegerät 10.

Die Faserzuführ-Einrichtung 1 besteht im wesentlichen aus zwei parallel angeordneten und gegeneinander verschiebbaren Glasplatten 11, welche eine Auflösung und Parallelisierung der Fasern 2 ermöglicht. Damit kann ein zwischen den beiden Glasplatten 11 liegender, endengeordneter Bart von Fasern 2 erzeugt werden. Die Einzelheiten dieser Faserzuführ-Einrichtung 1 sind in der Schweizer Patentanm. Nr. 02 438/89-9 beschrieben. Nach erfolgter Einführung der Fasern 2 in diese Einrichtung 1 liegen die Fasern 2 endengeordnet und parallel nebeneinander. Dieser Bart von Fasern 2 wird auf einen Tisch 8 gebracht, der in X- und Y-Richtung (Pfeile 12, 13) verschiebbar ist.
Der eigentliche Messaufbau besteht aus einem Stativ 6, an dem unten eine Beleuchtungsoptik 5 und oben eine Abbildungsoptik 3 befestigt sind. Zwischen diesen beiden Optiken 3,5 bewegt sich der Tisch 8 mit dem Faserbart. Auf der Abbildungsoptik 3 ist die CCD-Kamera 4 montiert.
Um eine möglichst grosse Schärfentiefe zu erreichen und damit die Messgenauigkeit möglichst unabhängig von der Fokussierung ist, weist die Abbildungsoptik 3 zusammen mit der Beleuchtungsoptik 5 einen telezentrischen Strahlengang auf. Unter einem telezentrischen Strahlengang durch ein optisches System versteht man solche optische Systeme, bei denen die Eintritts- oder die Austrittspupille im Unendlichen liegen.

Die Beleuchtungsoptik 5 ist vorzugsweise eine Hellfeldbeleuchtung, doch wäre auch eine Dunkelfeldbeleuchtung realisierbar. Weiter können auch Filter, wie z.B. Farbfilter oder Polarisationsfilter eingesetzt werden (speziell bei der Messung des Reifegrades von Fasern).

Die Grösse des Messfeldes 7 pro Bild ergibt sich aus der Pixelzahl des CCD-Sensors und der gewünschten Bildauflösung. Für eine Längenmessung reicht eine Auflösung von ca. 20 »m pro Pixel. Bei den heute-erhältlichen CCD-Sensoren mit 756 x 581 Square-Pixel ergibt sich ein Messfeld von 15,1 x 11,6 mm. Für die Messung der Feinheit muss eine Faserkante auf ca. 1 »m genau festgestellt werden, d.h. 1 »m pro Pixel, was einem Messfeld von 0,756 x 0,581 mm entspricht. Die Abbildungsoptiken 3 sind so konstruiert, dass das jeweilige Messfeld 7 auf dem ganzen CCD-Sensor 4 abgebildet wird.

Mit den erwähnten kleinen Messfeldern 7 kann natürlich nicht die ganze Faser 2 bzw. der ganze Faserbart erfasst werden. Deshalb werden mehrere Teilbilder von einem Faserbart aufgenommen. Der XY-Tisch 8 verschiebt den Faserbart nach jeder Aufnahme um eine Position weiter.

Im Rechner 9 werden die Teilbilder nach einem Algorhythmus, der auf die zu messenden Faserparameter abgestimmt ist, ausgewertet. Die Resultate der Teilbilder werden am Schluss der Messung zusammengesetzt und ergeben dann die Längenverteilung, Feinheitsverteilung, Reifegradverteilung oder Kräuselung, welche auf einem entsprechenden Monitor 10 graphisch dargestellt oder direkt zur Steuerung von faser- oder garnverarbeitenden Maschinen verwendet werden können.

Wenn mehrere, verschiedene Parameter gemessen werden sollen, die eine unterschiedliche Optik verlangen, so können mehrere Stative 6 mit entsprechenden Optiken 3,5 aufgebaut werden mit einer Transporteinheit für die Glasplatten 11 oder es können die verschiedenen Abbildungsoptiken 3 auf einem Drehteller - wie bei Mikroskopen üblich - montiert werden.

Der Auswertealgorhythmus der Bilder hängt vom parameterspezifischen internationalen Standard ab:
A) Längenmessung: Die Längenverteilung wird meistens nach Faserzahl oder nach Fasergewicht berechnet. Die Berechnung nach Faserzahl besteht dank den Möglichkeiten der Bilddatenverarbeitung aus dem Zählen der Fasern 2 und der Erfassung und Umrechnung der Position des Transporttisches 8 in mm. Bei der Berechnung nach Fasergewicht, wird jede gezählte Faser noch mit dem gemessenen Querschnitt gewichtet. Hier zeigt sich der Vorteil dieser Messmethode, denn jeder Faser kann die Länge und Dicke zugeordnet werden, wodurch sich eine korrekte Gewichtung ergibt. Bisher wurde mit Mittelwerten und Korrekturfaktoren gearbeitet.
B) Kräuselung: Die Fasern 2 liegen im gekräuselten Zustand zwischen den Glasplatten 11. Mit der Bilddatenverarbeitung können die Fasern 2 verfolgt werden und die Y- und X-Komponenten des Faserverlaufs addiert und miteinander verglichen werden. Der Abstand der Wendepunkte lässt sich natürlich auch feststellen, zusammen mit den X/Y-Komponenten ergibt sich ein Mass für die Kräuselung.
C) Feinheit: Für die Feinheit von runden Fasern 2, wie z.B. Wolle, wird die Breite gemessen und entsprechend umgerechnet. Die Baumwollfaser sieht aus wie ein spiralförmiges Bändchen. Durch diese Verdrehung des Bändchens ist einmal die Schmalseite und einmal die Breitseite der Faser 2 sichtbar. Beide lassen sich mit der Bilddatenverarbeitung messen. Durch eine Annäherungsformel kann daraus der Querschnitt und damit die Feinheit der Faser 2 berechnet werden.
D) Reifegrad: Die Baumwollfasern sind unterschiedlich reif. Ein Mass für den Reifegrad ist die Frequenz der Faserverdrehung, sowie das Verhältnis von Breit- und Schmalseite. Diese drei Parameter lassen sich mit dem erfindungsgemässen Verfahren ohne weiteres messen. Mit einer Farbkamera und einem Polarisationsfilter sowie einem Rotfilter kann der Reifegrad auch nach der Methode DIN 53943, Teil 3 gemessen werden.

## Patentansprüche

1. Verfahren zur Messung von quantitativen und qualitativen Eigenschaften von Fasern (2), insbesondere von Textilfasern, bei dem die zu messenden Fasern (2) oder Teilbereiche solcher Fasern (2) in planarer Anordnung zwischen eine Beleuchtungs- und eine Abbildungsoptik (5,3) gebracht und das durch die, senkrecht zur Faserebene stehende, Abbildungsoptik (3) erzeugte Bild von einem CCD-Sensor (4) aufgenommen und in einem Rechner (9) verarbeitet wird, dadurch gekennzeichnet, dass die Beleuchtungsoptik (5) und die Abbildungsoptik (3) einen telezentrischen Strahlengang aufweisen, der eine flächenhafte Durchstrahlung der Faserebene bewirkt.

2. Verfahren nach Anspruch 1, dadurch gekennzeichnet, dass die in planarer Anordnung vorliegenden, zu messenden Fasern (2) oder Teilbereiche solcher Fasern (2) schrittweise in x/y-Richtung verschoben werden um mehrere Messfelder (7) zu erzeugen.

3. Verfahren nach einem der Ansprüche 1 oder 2, dadurch gekennzeichnet, dass die zu messenden Fasern (2) endengeordnet sind.

4. Verfahren nach einem der Ansprüche 1 bis 3, dadurch gekennzeichnet, dass die zu messenden Fasern (2) mit monochromatischem Licht abgebildet werden.

5. Verfahren nach einem der Ansprüche 1 bis 4, dadurch gekennzeichnet, dass die zu messenden Fasern (2) mit polarisiertem Licht abgebildet werden.

6. Verfahren nach einem der Ansprüche 1 bis 5, dadurch gekennzeichnet, dass die zu messenden Fasern (2) in eine geordnete, vorzugsweise einlagige, planare Anordnung gebracht werden.

7. Vorrichtung zur Durchführung des Verfahrens nach einem der Ansprüche 1 bis 6, gekennzeichnet durch eine Einrichtung (1) zum Zuführen von Fasern (2) in planarer Anordnung zwischen eine Beleuchtungsoptik (5) und eine Abbildungsoptik (3), die einen telezentrischen Strahleugang aufweisen und deren optische Achse senkrecht auf der Faserebene steht, und durch einen CCD-Sensor (4) zur Umwandlung des von der Beleuchtungs- und Abbildungsoptik (5,3) erzeugten optischen Bildes in auswertbare, elektrische Signale.

8. Vorrichtung nach Anspruch 7, dadurch gekennzeichnet, dass die Beleuchtungsoptik (5) ein telezentrisches, F-Thetakorrigiertes Objektiv, das vorzugsweise katadioptrische Elemente aufweist, enthält.

9. Vorrichtung nach Anspruch 7 oder 8, gekennzeichnet durch einen in x/y-Richtung der Faserebene (12,13) verschiebbaren Tisch (8) zur Erzeugung mehrerer Messfelder (7).

10. Vorrichtung nach Anspruch 9, dadurch gekennzeichnet, dass die Abbildungsoptik (3) derart konstruiert ist, dass das jeweilige Messfeld (7) mit der verfügbaren Fläche des CCD-Sensors (4) übereinstimmt.

11. Vorrichtung nach einem der Ansprüche 7 bis 10, gekennzeichnet durch einen Rechner (9), der die elektrischen Signale auswertet.

12. Vorrichtung nach einem der Ansprüche 7 bis 11, gekennzeichnet durch ein optisches oder graphisches Anzeigegerät (10), zur benutzerorientierten Darstellung der gemessenen Fasereigenschaften.

13. Vorrichtung nach einem der Ansprüche 7 bis 12, dadurch gekennzeichnet, dass die Faserzuführ-Einrichtung (1) zwei parallel zueinander angeordnete und gegeneinander verschiebbare Glasplatten (11) umfasst.

## Claims

1. Method of measuring the quantitative and qualitative properties of fibres (2), in particular textile fibres, wherein the fibres (2) to be measured or areas of such fibres (2) are arranged in a planar manner between an illuminating and an imaging optic (5, 3) and the image generated by the imaging optic (3) located perpendicular to the fibre plane is received by a CCD sensor (4) and is processed in a computer (9), characterised in that the illuminating optic (5) and the imaging optic (3) have a telecentric ray path, so that the rays shine through the fibre plane in a planar manner.

2. Method according to claim 1, characterised in that the fibres (2) to be measured and presented in a planar arrangement or areas of such fibres (2) are displaced gradually in the x/y direction in order to produce a plurality of measuring fields (7).

3. Method according to either of claims 1 or 2, characterised in that the fibres (2) to be measured are arranged with their ends aligned.

4. Method according to one of claims 1 to 3, characterised in that the fibres (2) to be measured are imaged with monochromatic light.

5. Method according to one of claims 1 to 4, characterised in that the fibres (2) to be measured are imaged with polarised light.

6. Method according to one of claims 1 to 5, characterised in that the fibres (2) to be measured are arranged in a planar manner, preferably in a single layer.

7. Device for carrying out the method according to one of claims 1 to 6, characterised by a device (1) for feeding fibres (2) in a planar arrangement between an illuminating optic (5) and an imaging optic (3), which have a telecentric ray path and whose optical axis is perpendicular to the fibre plane, and by a CCD sensor (4) for converting the optical image generated by the illuminating and imaging optics (5, 3) into evaluatable electrical signals.

8. Device according to claim 7, characterised in that the illuminating optic (5) has a telecentric, F-theta-corrected objective, which preferably comprises catadioptric elements.

9. Device according to claim 7 or 8, characterised by a table (8) for generating a plurality of measuring fields (7) and displaceable in the direction of the fibre plane (12, 13).

10. Device according to claim 9, characterised in that the imaging optic (3) is so constructed that the respective measuring field (7) corresponds to the available area of the CCD sensor (4).

11. Device according to one of claims 7 to 10, characterised by a computer (9) for evaluating the electrical signals.

12. Device according to one of claims 7 to 11, characterised by an optical or graphic indicator (10) for indicating to the user the measured fibre properties.

13. Device according to one of claims 7 to 12, characterised in that the fibre feed device (1) comprises two glass plates (1) arranged parallel to one another and displaceable relative to one another.

## Revendications

1. Procédé pour la mesure de caractéristiques quantitatives et qualitatives de fibres (2), notamment de fibres textiles, dans lequel les fibres à mesurer (2) ou des zones partielles de telles fibres (2) sont amenées en une disposition plane entre des optiques d'éclairage (5) et de représentation (3) et l'image produite par l'optique de représentation (3) disposée perpendiculairement au plan de fibre est détectée par un capteur CCD (4) et est traitée dans un calculateur (9), caractérisé en ce que l'optique d'éclairage (5) et l'optique de représentation (3) présentent un trajet de rayons télécentrique qui provoque une irradiation de surface du plan des fibres.

2. Procédé selon la revendication 1, caractérisé en ce que les fibres (2) ou zones partielles de telles fibres (2) à mesurer, présentes suivant une disposition plane, sont déplacées pas-à-pas suivant la direction X/Y pour produire plusieurs champs de mesure (7).

3. Procédé selon l'une des revendications 1 ou 2, caractérisé en ce que les fibres à mesurer (2) présentent des bouts rangés.

4. Procédé selon l'une des revendications 1 à 3, caractérisé en ce que les fibres à mesurer (2) sont représentées par une lumière monochromatique.

5. Procédé selon l'une des revendications 1 à 4, caractérisé en ce que les fibres à mesurer (2) sont représentées par une lumière polarisée.

6. Procédé selon l'une des revendications 1 à 5, caractérisé en ce que les fibres à mesurer (2) sont amenées en une disposition plane rangée, de préférence en une couche.

7. Dispositif pour la mise en oeuvre du procédé selon l'une des revendications 1 à 6, caractérisé par un dispositif (1) pour l'amenée des fibres (2) en une disposition plane entre une optique d'éclairage (5) et une optique de représentation (3), qui présentent un trajet de rayons télécentrique et dont l'axe optique est orienté perpendiculairement au plan de fibres, et par un capteur CCD (4) pour transformer l'image optique produite par les optiques d'éclairage et de représentation (5, 3) en des signaux électriques exploitables.

8. Dispositif selon la revendication 7, caractérisé en ce que l'optique d'éclairage (5) comprend un objectif télécentrique, à correction F-thêta, qui présente de préférence des éléments catadioptriques.

9. Dispositif selon la revendication 7 ou 8, caractérisé par une table déplaçable suivant la direction X/Y du plan de fibres (12, 13) pour produire plusieurs champs de mesure (7).

10. Dispositif selon la revendication 9, caractérisé en ce que l'optique de représentation (3) est construite de façon que le champs de mesure respectif (7) coïncide avec la surface disponible du capteur CCD (4).

11. Dispositif selon l'une des revendications 7 à 10, caractérisé par un calculateur (9) qui exploite les signaux électriques.

12. Dispositif selon l'une des revendications 7 à 11, caractérisé par un appareil d'indication optique ou graphique (10) pour la représentation, conçue pour l'utilisateur, des caractéristiques de fibres mesurées.

13. Dispositif selon l'une des revendications 7 à 12, caractérisé en ce que le dispositif d'amenée de fibres (1) comprend deux plaques de verre (11) disposées parallèlement l'une à l'autre et déplaçables relativement l'une à l'autre.
